# EUROPÄISCHE PATENTANMELDUNG

(11) **EP 3 263 113 A1**
(43) Veröffentlichungstag der Anmeldung: **03.01.2018**
(21) Anmeldenummer: 17161379.7
(22) Anmeldetag: 16.03.2017
(51) Int. Cl.: A61K 31/593, A61K 31/202, A61K 36/16, A23L 33/105, A23L 33/12, A23L 33/155

(54) **ANWENDUNG UND PRÄPARAT (ENTHALTEND COLECALCIFEROL, OMEGA-3-FETTSÄURE AND GINKGO BILOBA) ZUR AUFRECHTERHALTUNG UND VERBESSERUNG KOGNITIVER FUNKTIONEN**

(30) Priorität: 30.06.2016 DE 102016111948
(71) Anmelder: Mertin, Jürgen, 88279 Amtzell (DE); Krumbholz, Rudolf, 66589 Merchweller (DE); Ruppert, Thomas, 66583 Spiesen-Elversberg (DE)
(72) Erfinder: Mertin, Jürgen, 88279 Amtzell (DE); Krumbholz, Rudolf, 66589 Merchweller (DE); Ruppert, Thomas, 66583 Spiesen-Elversberg (DE)
(74) Vertreter: Patentanwälte Bernhardt / Wolff Partnerschaft mbB

(57) **Zusammenfassung**

Die Erfindung betrifft eine Anwendung eines Präparats, das Colecalciferol, Omega-3-Fettsäure(n) und Extrakt aus Ginkgo biloba aufweist, zur Aufrechterhaltung und Verbesserung kognitiver Funktionen. In einer Ausgestaltung der Erfindung wird das das Präparat zur Verminderung von im Alterungsprozess auftretende Defiziten kognitiver Funktionen und zur Verbesserung und/oder Erhalt kognitiver Funktionen bei dementiellen Prozessen und/oder zur positiven Beeinflussung der kognitiven Leistungsfähigkeit des Gehirnes angewendet.

Die Erfindung betrifft ferner ein Präparat zur Aufrechterhaltung und Verbesserung kognitiver Funktionen, das als wirksame Bestandteile Colecalciferol, Omega-3-Fettsäure(n) und ein Extrakt von Ginkgo biloba aufweist. Zweckmäßigerweise umfassen die Omega-3-Fettsäure(n), vorzugsweise EPA, DHA, DPA, ETA, 21:5n3 und/oder SDA, und das Ginkgo biloba Extrakt ist aus Blättern des Ginkgo biloba gewonnen. In einer Ausführungsform der Erfindung weist das Präparat 800 bis 1200 Internationalen Einheiten Colecalciferol, 1 bis 4 g Omeag-3-Fettsäure(n) und 50 bis 300 mg Extrakt von Ginkgo biloba auf.

## Beschreibung

Die Erfindung betrifft die neue Anwendung eines kombinierten Wirkstoffpräparats als Nahrungsergänzungsmittel oder Medikament.

Im Rahmen des Alterungsprozesses treten bei vielen Menschen leichte kognitive Störungen auf, z.B. Beeinträchtigung der Gedächtnisfunktion, der Aufmerksamkeit sowie des Denkvermögens. Im höheren Alter können die Störungen stärker werden und Alltagsaktivitäten beeinträchtigen. Letzteres ist die Regel bei Erkrankungen des Gehirns mit zunehmendem dementiellen Prozess, bei der Alzheimerschen Erkrankung oder vaskulär verursachter Demenz.

Der Erfindung liegt die Aufgabe zugrunde, Beeinträchtigungen von kognitiven Funktionen im Alterungsprozess vorzubeugen.

Erfindungsgemäß wird diese Aufgabe gelöst durch die Anwendung des eingangs genannten Wirkstoffpräparats, das Colecalciferol, Omega-3-Fettsäure(n) und Extrakt aus Ginkgo biloba aufweist, zur Aufrechterhaltung und Verbesserung kognitiver Funktionen.

Durch die erfindungsgemäße Anwendung können im frühen Stadium der genannten Erkrankungen und bei kognitiven Störungen im Alter die zugrundeliegenden Schädigungen der Nervenzellen begrenzt und die Plastizität des Gehirns sowie seine Fähigkeit, neue Zellen zu generieren, unterstützt werden. Dies wirkt dem Verlust kognitiver Funktionen entgegen und kann im Falle dementieller Erkrankung zu einer Verlangsamung des klinischen Verlaufs führen und den Betroffenen ihre Selbständigkeit über längere Zeit erhalten.

Überraschend hat sich gezeigt, dass sich die jeweiligen unterschiedlichen Wirkweisen der Wirkstoffe im Gehirn derart ergänzen, dass das Präparat wirksamer ist als es die einzelne Einnahme der Wirkstoffe erwarten ließ.

In einer Ausführungsform der Erfindung wird das Präparat zur Verminderung von im Alterungsprozess auftretenden Defiziten kognitiver Funktionen und zur Verbesserung und/oder Erhalt kognitiver Funktionen bei dementiellen Prozessen angewendet.

Darüber hinaus kann das Präparat zur positiven Beeinflussung der kognitiven Leistungsfähigkeit des Gehirnes angewendet werden.

In einer Ausgestaltung der Erfindung wird das Präparat vorbeugend, vorzugsweise in primärer und/oder sekundärer Prophylaxe, zur Verbesserung und/oder dem Erhalt kognitiver Fähigkeiten angewendet.

Colecalciferol (Vitamin D₃) wird im menschlichen Körper über mehrere Zwischenschritte zum wirksamen Steroidhormon Calcitriol umgewandelt. Calcitriol bindet an einen Steroidrezeptor im Zellkern, den Vitamin D-Rezeptor (VDR), im Gehirn nachgewiesen u.a. im Hypothalamus, im Hippocampus und in dopaminergen Neuronen der Substania nigra. Der Ligand-Rezeptor-Komplex beeinflusst und steuert die Aktivität bestimmter Gene und damit die Synthese von Proteinen, die für die physiologische Regulation verschiedener Körperfunktionen benötigt werden.
Über die angeführten Wirkmechanismen induziert Colecalciferol in verschiedenen Hirnregionen die Bildung neurotropher Faktoren (nerve growth factor (NGF), brainderived neurotrophic factor (BDNF), glial cell line-derived neurotrophic factor (GDNF)), die für das Überleben und die Regeneration von Nervenzellen sowie die Neubildung solcher Zellen im Rahmen der lebenslang nachweisbaren Neuroplastizität von großer Bedeutung sind. Über nicht genomische Wirkmechanismen, nämlich membrangebundenen Steroidrezeptoren, reguliert und beeinflusst Colecalciferol die intrazelluläre Calcium-Homöostase, d.h. die Öffnung von Calciumkanälen, und hat eine neuroprotektive Funktion, indem es oxidativer Zellschädigung entgegenwirkt.

Veränderte Lebensbedingungen und Lebensgewohnheiten haben dazu geführt, dass in den westlichen Ländern ein Mangel an Vitamin D in der Bevölkerung weit verbreitet ist, wobei ältere Menschen besonders stark betroffen sind. Verantwortlich hierfür ist generell eine mangelnde Sonnenexposition. Darüber hinaus ist bei älteren Menschen die Fähigkeit vermindert, durch Sonneneinstrahlung Vitamin D zu produzieren. Eine Kompensierung der unzureichenden körpereigenen Produktion durch die Ernährung ist weitgehend eingeschränkt, da die herkömmlichen Lebensmittel sehr wenig oder gar kein Vitamin D enthalten. Lediglich mit fettreichem Fisch können nennenswerte Vitamin D Mengen zugeführt werden. Folglich kann die erfindungsgemäße Anwendung dem beschriebenen Mangel entgegenwirken.

Zweckmäßigerweise wird das Colecalciferol in dem Präparat in einer Dosierung von 800 - 1200 Internationalen Einheiten, vorzugsweise 1000 Internationalen Einheiten vorgesehen.

Mehrfach ungesättigte Fettsäuren haben Vitamincharakter und müssen mit der Nahrung zugeführt werden. Aus ihnen kann dann der menschliche Organismus durch Kettenverlängerung und durch Einfügen weiterer Doppelbindungen höher ungesättigte Fettsäuren herstellen. Dies geschieht über enzymatische Schritte, die in ihrer Kapazität begrenzt sind. So können höhere wirksame Konzentrationen im Körper nur durch die Einnahme hochungesättigter Fettsäuren, insbesondere von Eicosapentaensäure (EPA,C20:5) und Docosahexaensäure (DHA, C22:6), erreicht werden.

Höher ungesättigte Fettsäuren sind wesentliche Komponenten der Zellmembran und der weißen Substanz, des Myelins des Nervensystems. Zum anderen sind sie Vorläufersubstanzen für die Bildung wichtiger, für die Regulation vieler physiologischer Prozesse notwendiger Vermittlersubstanzen wie Prostaglandine, Leukotriene und Thromboxane. DHA wird benötigt für die Hirnentwicklung und die Funktion des Nervensystems. Erniedrigte DHA-Werte bewirken eine Schwächung kognitiver Funktionen und führen zu Verhaltensänderungen.
Wie Vitamin D haben Omega -3 Fettsäuren eine neuroprotektive Wirkung. Ebenso bewirken sie die Bildung neurotropher Faktoren wie BDNF, NGF, GDNF und fördern somit die Neuroplastizität, d.h. die Synaptogenese und das Entstehen neuer Neuronen.

Damit verbessern sie die Kognition und wirken der Entwicklung kognitiver Defizite und dementieller Prozesse entgegen.

Die kognitive Leistungsfähigkeit insbesondere die Gedächtnisfunktion und die Reaktionszeit kann durch Einnahme verbessert werden. Die im alternden Gehirn durch verschiedene Faktoren verursachte zunehmende neuronale Dysfunktion kann mit DHA aufgefangen werden, vor allem durch das DHA Derivat Neuroprotectin D1. Darüber hinaus wirkt DHA antientzündlich, da beim Hirnalterungsprozess und vor allem im Anfangsstadium dementieller Erkrankungen eine entzündliche Aktivierung der Microglia nachweisbar ist.

Das Präparat enthält die Omega-3-Fettsäure(n), vorzugsweise DHA und/oder EPA, in einer Ausführungsform der Erfindung in einer Dosierung von 1 - 4 g, vorzugsweise 1 -3g.
In einer Ausführungsform der Erfindung die umfassen die Omega-3-Fettsäure(n) EPA, DHA, DPA, ETA, 21:5n3 und/oder SDA. Die Omega-3-Fettsäure(n) liegen vorzugsweise mindestens in einem Anteil von 30 % als Monoglyceride vor, die der menschlichen Körper besonders gut aufnehmen kann.

Durch die Einnahme von Ginkgo biloba Extrakt wird die Durchblutung des Gehirns gefördert. Durch die daraus resultierende Verbesserung der Versorgung von Hirnzellen mit Sauerstoff und Glukose wird das Schwinden funktionstüchtiger Zellen vermindert und ihre Vernetzung und damit ihre Signalverarbeitung gefördert.

Das Ginkgo biloba ist in dem Präparat bevorzugt in einer Dosierung von 50 bis 300 mg, vorzugsweise 50 bis 100 mg, enthalten. Das Ginkgo biloba Extrakt ist zweckmäßigerweise ein aus Ginkgo Blättern hergestelltes Trockenextrakt, das als Trockenextrakt vorzugsweise 22 bis 27 % Flavonoide als Flavonglycoside (Mt 756.7), 2,6 bis 3,2 % Bilobalide, 2,8 bis 3,4 % Ginkgolide A, B und C sowie maximal 5 ppm Ginkgolsäure aufweist.

In einer Weiterbildung der Erfindung umfasst das Präparat als wirksamen Bestandteil Vitamin B, vorzugsweise Vitamin B6, Vitamin B9 und/oder Vitamin B12, wobei das Präparat bevorzugt die Vitamin B enthaltende Kapseln bzw. Granulatkörnchen aufweist. In einer Ausführungsform der Erfindung weist das Präparat 0,195 bis 10 mg Vitamin B6, 0,045 bis 0,5 mg Vitamin B9 und/oder 0,375 bis 210 µg Vitamin B12 auf.

Vorzugsweise wird das Präparat einmal täglich per os eingenommen.

In einer bevorzugten Ausgestaltung der Erfindung ist das Präparat durch eine Vielzahl von, bevorzugt kugelförmigen, Kapseln und/oder Granulatkörnern gebildet, die jeweils einen maximalen Durchmesser von 3 mm, vorzugsweise von 2 mm, aufweisen. Zweckmäßigerweist ist das Gingko Biloba Extrakt und/oder das Vitamin B in den Granulatkörnern enthalten. Das Colecaliciferol und die Omega-3-Fettsäure(n) und ggf. das Vitamin B sind vorzugsweise gemeinsam in den Kapseln enthalten. Vorstellbar wäre aber auch, für jeden der Wirkstoffe eigene Kapseln bzw. Granulate, d.h. unterschiedliche Kapseln bzw. Granulatkörner für das Colecaliciferol, die Omega-3-Fettsäure(n), das Gingko biloba Extrakt und/oder das Vitamin B, vorzusehen.

### 1. Beispiel:

Ein erfindungsgemäßes Präparat umfasst in seiner Darreichungsform einen Einweg-Behälter, z.B. ein Papiertütchen, in dem folgende zur peroralen Einnahme einmal täglich vorgesehenen Komponenten enthalten sind:
- eine Vielzahl von kugelrunden Kapseln, die einen Durchmesser von 2 mm aufweisen und in ihrer Gesamtheit als wirksame Bestandteile sowohl 1000 Internationale Einheiten Colecalciferol als auch 1 g DHA enthalten.
- eine Vielzahl von Granulatkörnern, die einen maxiamalen Durchmesser von 2 mm aufweisen und in ihrer Gesamtheit als wirksamen Bestandteil 75 mg Trockenextrakt von Gingko Biloba enthalten.

### 2. Beispiel:

Ein erfindungsgemäßes Präparat umfasst in seiner Darreichungsform einen Einweg-Behälter, z.B. ein Papiertütchen, in dem folgende zur peroralen Einnahme einmal täglich vorgesehenen Komponenten enthalten sind:
- eine Vielzahl von kugelrunden Kapseln, die einen Durchmesser von 2 mm aufweisen und in ihrer Gesamtheit als wirksame Bestandteile sowohl 1000 Internationale Einheiten Colecalciferol als auch 1,1 g DHA enthalten.
- eine Vielzahl von Granulatkörnern, die einen maximalen Durchmesser von 2 mm aufweisen und in ihrer Gesamtheit als wirksamen Bestandteil 95 mg Trockenextrakt von Gingko Biloba enthalten.
- eine Vielzahl von Kapseln oder Granulatkörnern, die einen maximalen Durchmesser von 2 mm aufweisen und in ihrer Gesamtheit als wirksamen Bestandteil 1 µg Vitamin B12 aufweisen.

### 3. Beispiel:

Ein erfindungsgemäßes Präparat umfasst in seiner Darreichungsform einen Einweg-Behälter, z.B. ein Papiertütchen, in dem folgende zur peroralen Einnahme einmal täglich vorgesehenen Komponenten enthalten sind:
- eine Vielzahl von kugelrunden Kapseln, die einen Durchmesser von 2,5 mm aufweisen und in ihrer Gesamtheit als wirksame Bestandteile sowohl 1100 Internationale Einheiten Colecalciferol als auch 1,5 g DHA enthalten.
- eine Vielzahl von Granulatkörnern, die einen maximalen Durchmesser von 2 mm aufweisen und in ihrer Gesamtheit als wirksamen Bestandteil 80 mg Trockenextrakt von Gingko Biloba enthalten.

### 4. Beispiel:

Ein erfindungsgemäßes Präparat umfasst in seiner Darreichungsform einen Einweg-Behälter, z.B. ein Papiertütchen, in dem folgende zur peroralen Einnahme einmal täglich vorgesehene Komponenten enthalten sind:
- eine Vielzahl von kugelrunden Kapseln, die einen Durchmesser von 2,5 mm aufweisen und in ihrer Gesamtheit als wirksame Bestandteile sowohl 1200 Internationale Einheiten Colecalciferol als auch 2 g DHA enthalten.
- eine Vielzahl von Granulatkörnern, die einen maximalen Durchmesser von 2 mm aufweisen und in ihrer Gesamtheit als wirksamen Bestandteil 80 mg Trockenextrakt von Gingko Biloba enthalten.
- eine Vielzahl von kugelrunden Kapseln oder Granulatkörnern, die einen maximalen Durchmesser von 2 mm aufweisen und in ihrer Gesamtheit als wirksamen Bestandteil 1 µg Vitamin B12 aufweisen.

## Patentansprüche

1. Anwendung eines Präparats, das Colecalciferol, Omega-3-Fettsäure(n) und Extrakt aus Ginkgo biloba aufweist, zur Aufrechterhaltung und Verbesserung kognitiver Funktionen.

2. Anwendung nach Anspruch 1,
**dadurch gekennzeichnet,**
**dass** das Präparat zur Verminderung von im Alterungsprozess auftretende Defiziten kognitiver Funktionen und zur Verbesserung und/oder Erhalt kognitiver Funktionen bei dementiellen Prozessen angewendet wird.

3. Anwendung nach Anspruch 1 und 2,
**dadurch gekennzeichnet,**
**dass** das Präparat zur positiven Beeinflussung der kognitiven Leistungsfähigkeit des Gehirns angewendet wird.

4. Anwendung nach einem der Ansprüche 1 bis 3,
**dadurch gekennzeichnet,**
**dass** das Präparat vorbeugend, vorzugsweise in primärer und/oder sekundärer Prophylaxe, zur Verbesserung und/oder dem Erhalt kognitiver Fähigkeiten angewendet wird.

5. Anwendung nach den Ansprüchen 1 bis 4,
**dadurch gekennzeichnet,**
**dass** das Colecalciferol in einer Dosierung von 800 - 1200 Internationalen Einheiten, vorzugsweise 1000 Internationalen Einheiten, die Omega-3-Fett-säure(n), vorzugsweise DHA und/oder EPA, in einer Dosierung von 1 - 4 g, vorzugsweise 1 - 3 g, und das Ginkgo biloba Extrakt in einer Dosierung von 50 - 300 mg, vorzugsweise 50 - 100 mg, im Präparat gemeinsam verabreicht werden.

6. Anwendung nach einem der Ansprüche 1 bis 5,
**dadurch gekennzeichnet,**
**dass** das Präparat einmal täglich per os eingenommen wird.

7. Anwendung nach einem der Ansprüche 1 bis 6,
**dadurch gekennzeichnet,**
**dass** das Präparat in einer Vielzahl von, vorzugsweise kugelförmigen Kapseln und/oder Granulatkörnern verabreicht wird, die einen maximalen Durchmesser von 3 mm, vorzugsweise von 2 mm, aufweisen, wobei
das Gingko Biloba und/oder das Vitamin B vorzugsweise in den Granulatkörnern enthalten ist, und das Colecaliciferol und die Omega-3-Fettsäure(n), ggf. gemeinsam, in den Kapseln enthalten.

8. Präparat zur Aufrechterhaltung und Verbesserung kognitiver Funktionen, das als wirksame Bestandteile Colecalciferol, Omega-3-Fettsäure(n) und ein Extrakt von Ginkgo biloba aufweist.

9. Präparat nach Anspruch 8,
**dadurch gekennzeichnet,**
**dass** die Omega-3-Fettsäure(n) EPA, DHA, DPA, ETA, 21:5n3 und/oder SDA umfassen, wobei die Omega-3-Fettsäure(n) vorzugsweise mindestens in einem Anteil von 30 % als Monoglyceride vorliegen.

10. Präparat nach Anspruch 8 oder 9,
**dadurch gekennzeichnet,**
**dass** das Ginkgo biloba Extrakt aus Blättern des Ginkgo biloba gewonnen ist.

11. Präparat nach einem der Ansprüche 8 bis 10,
**dadurch gekennzeichnet,**
**dass** das Präparat die wirksamen Bestandteile in folgenden Mengen enthält:
- Colecalciferol: 800 bis 1200 Internationalen Einheiten, vorzugsweise 1000 Internationalen Einheiten,
- Omeag-3-Fettsäure(n): 1 bis 4 g, vorzugsweise 1 bis 3 g, und
- Extrakt von Ginkgo biloba: 50 bis 100 mg.

12. Präparat nach einem der Ansprüche 8 bis 11,
**dadurch gekennzeichnet,**
**dass** das Präparat durch eine Vielzahl von, vorzugsweise kugelförmigen, Kapseln und/oder Granulatkörnern gebildet ist, die einen maximalen Durchmesser von 3 mm, vorzugsweise von 2 mm, aufweisen, wobei das Gingko Biloba und/oder das Vitamin B vorzugsweise in den Granulatkörnern enthalten ist, und das Colecaliciferol und die Omega-3-Fettsäure(n), ggf. gemeinsam, in den Kapseln enthalten.

13. Präparat nach einem der Ansprüche 8 bis 12,
**dadurch gekennzeichnet,**
**dass** das Präparat als wirksamen Bestandteil Vitamin B, vorzugsweise Vitamin B6, Vitamin B9 und/oder Vitamin B12, aufweist, wobei für das Präparat bevorzugt weitere Kapseln und/oder Granulatkörner vorgesehen sind, die das Vitamin B enthalten.
